(19) Europäisches Patentamt
European Patent Office
Office européen des brevets

(11) Veröffentlichungsnummer: **0 300 162**
**A2**

# (12) EUROPÄISCHE PATENTANMELDUNG

(21) Anmeldenummer: **88108297.8**

(22) Anmeldetag: **25.05.88**

(51) Int. Cl.4: **C07C 67/04 , C07C 69/14 , C07C 69/24 , C07C 69/78 , B01J 31/16**

(30) Priorität: **18.07.87 DE 3723891**

(43) Veröffentlichungstag der Anmeldung: **25.01.89 Patentblatt 89/04**

(84) Benannte Vertragsstaaten: **BE CH DE ES FR GB IT LI NL**

(71) Anmelder: **HÜLS AKTIENGESELLSCHAFT**
**Patentabteilung / PB 15 - Postfach 13 20**
**D-4370 Marl 1(DE)**

(72) Erfinder: **Gude, Fritz, Dr.**
**Zur Emschermulde 24**
**D-4690 Herne 2(DE)**
Erfinder: **Bellut, Hans, Dr.**
**Bergstrasse 50**
**D-4408 Dülmen(DE)**

## (54) Verfahren zur Herstellung von Dicyclopentenolestern.

(57) Es bestand die Aufgabe, die an sich mittels saurer Katalysatoren bekannte, aber in bezug auf Aufarbeitung sehr aufwendige Herstellung der Ester zu vereinfachen.

Gelöst wurde die Aufgabe durch Einsatz einer leicht flüchtigen $BF_3$-Additionsverbindung als Katalysator. Umsetzung und Isolierung erfolgen in einem Eintopfverfahren.

Die Produkte finden Verwendung als Riechstoffe.

EP 0 300 162 A2

## Verfahren zur Herstellung von Dicyclopentenolestern

Die vorliegende Erfindung betrifft ein Verfahren zur Herstellung von Dicyclopentenolestern aus Dicyclopentadien und organischen Carbonsäuren unter Einwirkung saurer Lewis-Katalysatoren.

Die nachfolgend näher beschriebene Umsetzung und die Isolierung des Produkts erfolgt in einem Eintopfverfahren ohne zusätzliche aufwendige Wasch- oder Filtrierschritte.

Zur Herstellung von Dicyclopentenolestern nachstehend allgemeiner Formel

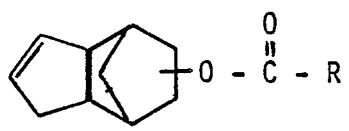

R = Alkyl mit 1 bis 12 C-Atomen, Aryl und subst. Aryl

sind grundsätzlich verschiedene Verfahren bekannt, die von den gleichen Ausgangsstoffen - Cyclopentadien und organischen Säuren - ausgehen, aber zur Aufarbeitung kostenintensive, aufwendige Arbeitsschritte benötigen. So müssen bei Verwendung fester Katalysatoren Filtration, bei Verwendung flüssiger Katalysatoren Neutralisation und wäßrige Extraktion in Kauf genommen werden. Normalerweise muß mit einem Totalverlust des angewandten Katalysatorsystems gerechnet werden, wodurch unter anderem zusätzliche Entsorgungs- und Aufarbeitungsprobleme entstehen. Durch diese zusätzlich anfallenden Verfahrensschritte wird das Endprodukt preislich stark belastet; deshalb wurde nach einem einfacheren Verfahren gesucht.

Alle bekannten Verfahren arbeiten nach der Methode, daß die verwendeten Katalysatoren vor der Aufarbeitung entweder zerstört oder auf anderem Wege aus dem System entfernt werden, um einerseits die Rückspaltung der Ester in die Ausgangsstoffe und andererseits eine weitergehende Verharzung des Reaktionsproduktes zu vermeiden.

Es ist weiterhin bekannt, daß unter zahlreichen Typen von Lewis-Säuren wie Schwefelsäure, Perchlorsäure, organische Sulfonsäuren auch Borfluorid bzw. Borfluorid-Phosphorsäure als Veresterungskatalysatoren benutzt werden können (US-PS 2 457 157). Borfluorid hat jedoch zusätzlich die unangenehme Eigenschaft, die Oligomerisation olefinischer Verbindungen zu katalysieren (vgl. H. S. Booth und D. R. Martin in Boron Trifluoride and its Derivates, John Wiley & Sons, Inc., 1949), was im Falle der Dicyclopentenolester zu vermehrter Harzbildung führen kann.

Überraschend wurde nun gefunden, daß die leichter flüchtigen Additionsverbindungen des Borfluorids, wie $BF_3$-Essigsäure, $BF_3$-Propionsäure, $BF_3$ Methylether, $BF_3$-Ethylether, $BF_3$-Methylether, $BF_3$-Alkohole, wie $BF_3$-Ethanol und ähnliche die Anlagerung von Carbonsäuren an Olefine, wie zum Beispiel an Dicyclopentadien zwar katalysieren, die Verharzung jedoch bei Einhaltung günstiger Konzentrationen nicht merklich fördern. $BF_3$-Additionsverbindungen eignen sich deshalb ausgezeichnet als Hilfsmittel für die beschriebene Additionsreaktion von Carbonsäuren an Dicyclopentadien.

Gegenstand der Erfindung ist daher ein Verfahren zur Herstellung von Dicyclopentenolestern der allgemeinen Formel

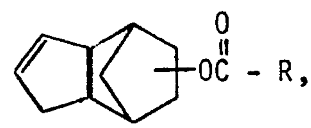

worin R = Alkyl mit 1 bis 12 C-Atomen, Aryl bzw. substituiertes Aryl bedeutet, aus Dicyclopentadien und den entsprechenden organischen Carbonsäuren und in Gegenwart von Lewissäure-Katalysatoren, welches dadurch gekennzeichnet ist, daß als Katalysatoren leichtflüchtige Additionsverbindungen von $BF_3$ eingesetzt werden.

Die Arylverbindungen können z. B. Phenyl-, Tolyl-, Chlor-Phenyl, Naphthyl-Verbindungen usw. sein.

Die Zerstörung bzw. gesonderte Abtrennung des Katalysators vor der Aufarbeitung erweist sich als überflüssig, da er bei der destillativen Reinigung leicht als getrennte Fraktion isoliert und erneut eingesetzt werden kann. Die Rückspaltung der gebildeten Ester läßt sich bei den angewandten Bedingungen vernachlässigen. Auf diese Weise erreicht man ein kostengünstiges, umweltfreundliches Verfahren zur Herstellung von Dicyclopentenolestern.

Zur Herstellung der gewünschten Dicyclopentenolester versetzt man handelsübliches Dicyclopentadien ohne Vorbehandlung mit der 1 bis 5fachen Menge Carbonsäure, fügt 0,1 bis 15 %, am günstigsten jedoch 0,5 bis 2 % einer $BF_3$-Additionsverbindung, bezogen auf Dicyclopentadien zu und kocht bis zur Beendigung der Reaktion etwa 1/4 bis 2 Stunden unter Rückfluß. Danach wird sofort im Vakuum destilliert. Der Vorlauf - Carbonsäureüberschuß und $BF_3$-Additionsverbindungen - findet ohne weitere Behandlung nach Ersatz der verbrauchten bzw. verlorenen Mengen für die nachfolgende Charge Verwendung. Den als Hauptfraktion übergehenden Ester leitet man über eine Waschsäule aus Marmor/Aktivkohle, um noch

eventuell vorhandene freie Säurespuren, Katalysatorreste und kleine Oligomerenanteile zu binden. Das gewonnene Produkt besitzt danach die geforderte Spezifikation. Als Gefäßmaterial für die thermisch belasteten Apparateteile eignen sich übliche austenitische Stähle, zum Beispiel vom Typ V4A. Die erzielbaren Ausbeuten liegen im Bereich 80 bis 85 %, bezogen auf eingesetztes Rein-Dicyclopentadien. Durch schärferes Ausdestillieren lassen sich noch weitere 5 bis 10 % als Nachlauf gewinnen, doch müssen diese Mengen einerseits mit zusätzlichem Aufwand gereinigt werden, andererseits wird der Rückstand so zähflüssig, daß die Entleerung der Destillierblase Schwierigkeiten bereitet. Der Rückstand ist mit ca. 500 ppm praktisch katalysatorfrei und kann ohne besondere Vorkehrungen durch Verbrennung entsorgt werden.

Die Cyclopentenolester finden Anwendung als Riechstoffe.

Beispiel 1

132 g (1,0 Mol) technisches Dicyclopentadien mit 93 % Gehalt (gaschromatografisch) an reinem Dicyclopentadien werden mit 120 g (2,0 Mol) Eisessig vermischt. Nach Zufügen von 2 g (0,01 Mol) $BF_3 \cdot 2\ CH_3COOH$ wird unter Rühren 1 Stunde am Rückfluß erhitzt, dabei steigt die Temperatur von anfangs 118 °C auf 140 °C. Anschließend wird destilliert.

1) $Kp_{75}$ = 58 °C; 66 g (Essigsäure)

2) $Kp_{15}$ = ≤ 124 °C, 14 g ($BF_3 \cdot$ 2 Essigsäure, Essigsäure, Dicyclopentenolacetat)

3) $Kp_{15}$ = 126 °C; 140 g (Dicyclopentenolacetat)

4) 31 g (Rückstand) mit C 80,3;H 8,7; O 10,6; F 700 pm

Fraktion 3) wird über eine 60 cm lange und 2 cm breite mit Marmor/Aktivkohle gefüllte Säule geleitet. Danach werden 140 g entsprechend 78,4 %, bezogen auf Rein-Dicyclopentadien mit $n_D^{20}$ = 1,4980 einer Säurezahl SZ = 0,24 und mit 98,9 % Reinheit GC erhalten.

Beispiel 2

Fraktion 1) und Fraktion 2) aus Beispiel 1 werden mit 54 g Eisessig und 0,5 g $BF_3 \cdot 2\ CH_3COOH$ versetzt und analog Beispiel 1 umgesetzt und aufgearbeitet.

Ausbeute 146 g entsprechend 81,8 % Dicyclopentenolacetat mit den gleichen Daten wie im Beispiel 1 angeführt.

Beispiel 3

132 g (1,0 Mol) technisches Dicyclopentadien werden mit 148 g (2,0 Mol) Propionsäure unter Zusatz von 1,2 g (0,01 Mol) $BF_3 \cdot O(CH_3)_2$ wie im Beispiel 1 eingesetzt und aufgearbeitet.

1) $Kp_{75}$ = 85 °C; 91 g (Propionsäure)

2) $Kp_{15}$ = ≤ 138 °C; 15 g ($BF_3$-Additionsverbindung und Übergänge)

3) $Kp_{15}$ = 140 °C; 143 g (Dicyclopentenolpropionat)

4) 28 g (Rückstand) mit C 81,28; H 8,92; O 7,90; F 800 ppm

Nach der Reinigung über Marmor/Aktiv-Kohle werden 143 g entsprechend 74,6 % mit $n_D^{20}$ = 1,4942, SZ = 0,16 und 99,2 % Reinheit erhalten.

Beispiel 4

Analog Beispiel 2 werden die Vorfraktionen aus Beispiel 3 in einem Rückführungsversuch verwertet.

Ausbeute 163 g entsprechend 85,0 % Dicyclopentenolpropionat gleicher Qualität wie im Beispiel 3.

Beispiel 5

264 g (2,0 Mol) technisches Dicyclopentadien mit 93 % Gehalt (gaschromatografisch) an reinem Dicyclopentadien werden mit 0,62 g (0,01 Mol) $BF_3 \cdot O(CH_3)_2$ versetzt und auf 130 °C erhitzt. Unter Rühren wird dann während einer halben Stunde 122 g (1,0 Mol) Benzoesäure portionsweise eingetragen. Dabei steigt die Reaktionstemperatur auf 155 °C. Diese Temperatur wird zur Nachreaktion 1 Stunde gehalten, danach läßt man abkühlen. Zur Aufarbeitung wird im Vakuum destilliert.

1) $Kp_3$ = 50 bis 115 °C; 90 g ($BF_3 \cdot O(CH_3)_2$; DCP)

2) $Kp_4$ = ≤ 180 °C; 27 g (Benzoesäure, DCP-Benzoat)

3) $Kp_4$ = 182 °C; 200 g (DCP-Benzoat)

4) 59 g (Rückstand)

Fraktion 3) läßt sich bei $Kp_1$ = 150 °C praktisch rückstandsfrei als hellgelbe, leicht ölige Flüssigkeit redestillieren mit $n_D^{20}$ = 1,5573 einer Säurezahl SZ = 0,1 und mit 92,4 % Reinheit GC.

**Ansprüche**

1. Verfahren zur Herstellung von Dicyclopentenolestern der allgemeinen Formel

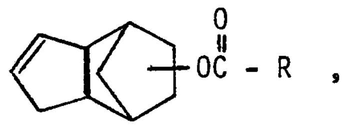

worin R = Alkyl mit 1 bis 12 C-Atomen, Aryl bzw. substituiertes Aryl bedeutet,
aus Dicyclopentadien und den entsprechenden organischen Carbonsäuren und in Gegenwart von Lewissäure-Katalysatoren,
dadurch gekennzeichnet,
daß als Katalysatoren leichtflüchtige Additionsverbindungen von $BF_3$ eingesetzt werden.

2. Verfahren nach Anspruch 1,
dadurch gekennzeichnet,
daß die Additionsverbindungen von $BF_3$ mit $C_2$- bis $C_3$-Carbonsäuren eingesetzt werden.

3. Verfahren nach Anspruch 1,
dadurch gekennzeichnet,
daß die Additionsverbindungen von $BF_3$ mit Methanol oder Ethanol eingesetzt werden.

4. Verfahren nach Anspruch 1,
dadurch gekennzeichnet,
daß die Additionsverbindungen von $BF_3$ Dimethyl-/Diethyl- oder Methyl-Ethyl-Ethern eingesetzt werden.

5. Verfahren nach Anspruch 1,
dadurch gekennzeichnet,
daß der Katalysator aus $BF_3$-Additionsverbindungen als Vorlauf zurückgewonnen wird und ohne weitere Behandlung erneut einsetzbar ist.